# EUROPEAN PATENT SPECIFICATION

(11) **EP 3 656 792 B1**
(45) Date of publication and mention of the grant of the patent: **03.07.2024**
(21) Application number: 20150344.8
(22) Date of filing: 28.12.2012
(51) Int. Cl.: C07K 16/00, A61K 38/26

(54) **IMMUNOGLOBULIN FC VARIANTS**
IMMUNGLOBULIN-FC-VARIANTEN
VARIANTES FC D'IMMUNOGLOBULINE

(30) Priority: 30.12.2011 KR 20110147683
(43) Date of publication of application: 27.05.2020
(62) Divisional of application: 12861250.4
(73) Proprietor: Hanmi Science Co., Ltd., Hwaseong-si, Gyeonggi-do 445-813 (KR)
(72) Inventor: OH, Euh Lim, 134-734 Seoul (KR); HUH, Yong Ho, 152-772 Seoul (KR); HWANG, Sang Youn, 445-769 Hwaseong-si Gyeonggi-do (KR); CHOI, In Young, 448-755 Yongin-si Gyeonggi-do (KR); JUNG, Sung Youb, 442-190 Suwon-si Gyeonggi-do (KR); KWON, Se Chang, 135-506 Seoul (KR)
(74) Representative: Mewburn Ellis LLP

(56) References cited:
- EP-A1- 2 233 500
- WO-A1-2010/045193
- WO-A2-2006/130834
- WO-A2-2011/122011
- WO-A2-2013/004842
- US-A1- 2005 014 934
- US-A1- 2007 135 620
- US-A1- 2009 041 770
- YEUNG Y A ET AL: "A Therapeutic Anti-VEGF Antibody with Increased Potency Independent of Pharmacokinetic Half-life", CANCER RESEARCH, AMERICAN ASSOCIATION FOR CANCER RESEARCH, US, vol. 70, no. 8, 15 April 2010 (2010-04-15) , pages 3269-3277, XP002738426, ISSN: 0008-5472, DOI: 10.1158/0008-5472.CAN-09-4580 [retrieved on 2010-03-30]
- R. F. LATYPOV ET AL: "Elucidation of Acid-induced Unfolding and Aggregation of Human Immunoglobulin IgG1 and IgG2 Fc", JOURNAL OF BIOLOGICAL CHEMISTRY, vol. 287, no. 2, 6 January 2012 (2012-01-06), pages 1381-1396, XP055084668, ISSN: 0021-9258, DOI: 10.1074/jbc.M111.297697
- SHIELDS R L ET AL: "High resolution mapping of the binding site on human IgG1 for FcgammaRI, FcgammaRII, FcgammaRIII, and FcRn and design of IgG1 variants with improved binding to the FcgammaR", JOURNAL OF BIOLOGICAL CHEMISTRY, AMERICAN SOCIETY FOR BIOCHEMISTRY AND MOLECULAR BIOLOGY, US, vol. 276, no. 9, 2 March 2001 (2001-03-02) , pages 6591-6604, XP002271092, ISSN: 0021-9258, DOI: 10.1074/JBC.M009483200
- PETKOVA STEFKA B ET AL: "Enhanced half-life of genetically engineered human IgG1 antibodies in a humanized FcRn mouse model: potential application in humorally mediated autoimmune disease", INTERNATIONAL IMMUNOLOGY, OXFORD UNIVERSITY PRESS, GB, vol. 18, no. 12, 1 December 2006 (2006-12-01), pages 1759-1769, XP002539987, ISSN: 0953-8178, DOI: 10.1093/INTIMM/DXL110
- JONATHAN ZALEVSKY ET AL: "Enhanced antibody half-life improves in vivo activity", NATURE BIOTECHNOLOGY, vol. 28, no. 2, 1 February 2010 (2010-02-01), pages 157-159, XP055049187, ISSN: 1087-0156, DOI: 10.1038/nbt.1601
- HINTON P R ET AL: "Engineered human IgG antibodies with longer serum half-lives in primates", JOURNAL OF BIOLOGICAL CHEMISTRY, AMERICAN SOCIETY FOR BIOCHEMISTRY AND MOLECULAR BIOLOGY, US, vol. 279, no. 8, 20 February 2004 (2004-02-20), pages 6213-6216, XP002305813, ISSN: 0021-9258, DOI: 10.1074/JBC.C300470200
- YEUNG YIK ANDY ET AL: "Engineering Human IgG1 Affinity to Human Neonatal Fc Receptor: Impact of Affinity Improvement on Pharmacokinetics in Primates", THE JOURNAL OF IMMUNOLOGY, THE AMERICAN ASSOCIATION OF IMMUNOLOGISTS, US, vol. 182, no. 12, 1 June 2009 (2009-06-01) , pages 7663-7671, XP002566420, ISSN: 0022-1767, DOI: 10.4049/JIMMUNOL.0804182
- CARLOS VACCARO ET AL: "Engineering the Fc region of immunoglobulin G to modulate in vivo antibody levels", NATURE BIOTECHNOLOGY, vol. 23, no. 10, 1 October 2005 (2005-10-01), pages 1283-1288, XP055049342, ISSN: 1087-0156, DOI: 10.1038/nbt1143
- Randall J. Brezski and Juan Carlos Almagro: "Development of Antibody-Based Therapeutics: Translational Considerations" In: Mohammad A. Tabrizi, Gadi G. Bornstein, Scott L. Klakamp: "Application of antibody engineering", 24 April 2012 (2012-04-24), Springer, XP009187858, ISBN: 9781441959553 pages 65-93, * The whole document, in particular, Table 4.1 *
- BURMEISTER W P ET AL: "CRYSTAL STRUCTURE OF THE COMPLEX OF RAT NEONATAL FC RECEPTOR WITH FC", NATURE, MACMILLAN JOURNALS LTD, LONDON, vol. 372, no. 6504, 24 November 1994 (1994-11-24), pages 379-383, XP000876647, ISSN: 0028-0836, DOI: 10.1038/372379A0
- JONATHAN ZALEVSKY ET AL: "Enhanced antibody half-life improves in vivo activity", NATURE BIOTECHNOLOGY, vol. 28, no. 2, 1 February 2010 (2010-02-01), pages 157-159, XP55395562, us ISSN: 1087-0156, DOI: 10.1038/nbt.1601
- CHEN YANG ET AL: "Evaluation of a Catenary PBPK Model for Predicting theIn VivoDisposition of mAbs Engineered for High-Affinity Binding to FcRn", THE AAPS JOURNAL, SPRINGER US, BOSTON, vol. 14, no. 4, 7 September 2012 (2012-09-07), pages 850-859, XP035719313, DOI: 10.1208/S12248-012-9395-9 [retrieved on 2012-09-07]
- ALAIN BECK ET AL: "Therapeutic Fc-fusion proteins and peptides as successful alternatives to antibodies", MABS, vol. 3, no. 5, 1 September 2011 (2011-09-01), pages 415-416, XP55268639, US ISSN: 1942-0862, DOI: 10.4161/mabs.3.5.17334
- STROHL ET AL: "Optimization of Fc-mediated effector functions of monoclonal antibodies", CURRENT OPINION IN BIOTECHNOLOGY, LONDON, GB, vol. 20, no. 6, 1 December 2009 (2009-12-01), pages 685-691, XP026778879, ISSN: 0958-1669, DOI: 10.1016/J.COPBIO.2009.10.011 [retrieved on 2009-11-04]
- T. Igawa ET AL: "Reduced elimination of IgG antibodies by engineering the variable region", Protein Engineering Design and Selection, vol. 23, no. 5, 1 May 2010 (2010-05-01), pages 385-392, XP055003115, ISSN: 1741-0126, DOI: 10.1093/protein/gzq009

## Description

### [Technical Field]

The present disclosure relates to immunoglobulin Fc variants having an increased binding affinity for FcRn (neonatal Fc receptor) and a method for increasing *in vivo* half-life of a physiologically active polypeptide using the same. The immunoglobulin Fc variants are characterized by including one or more amino acid modifications selected from the group consisting of 307S, 308F, 380S, 380A, 428L, 429K, 430S, 433K and 434S (this numbering is according to the EU index) in the constant region of a native immunoglobulin Fc fragment. The present invention relates to protein conjugates in which a physiologically active polypeptide is covalently linked to an immunoglobulin Fc variant via a polyethylene glycol.

### [Background Art]

An antibody is an immune protein that binds to a particular antigen. An antibody is composed of two light polypeptide chains and two heavy polypeptide chains. Each chain is composed of immunoglobulin domains and has both a variable region and a constant region. The variable regions show significant sequence diversity between the antibodies and are responsible for binding to the target antigen. The constant regions, having relatively low sequence diversity, are responsible for binding to a number of natural proteins and elicit important biochemical events.

Detailed descriptions of the structures, functions and subclasses of antibodies are disclosed in a lot of previous literature (Burton DR: Immunoglobulin G: functional sites, Mol. Immunol. 22: 161-206, 1985). The region between the Fc domains in IgG mediates interaction with the neonatal Fc receptor (FcRn). FcRn catches IgG that enters the cell by endocytosis and recycles it from the endosome back to the bloodstream (Raghavan et al., Annu. Rev. Cell Dev. Biol. 12: 181-220, 1996). With the exclusion of kidney filtration due to the large size of the antibody itself, this process results in prolonged antibody serum half-life ranging from one to three weeks.

Studies of rat and human Fc domains have demonstrated the importance of some Fc residues to the FcRn binding. In the murine Fcγ domain, random mutation and phage display selection at the sites T252, T254, and T256 lead to a triple mutant T252L/T254S/T256F that has increased FcRn binding affinity and serum half-life (Ghetie et al., Nat. Biotech. 15(7): 637-640, 1997). Disruption of the Fc/FcRn interaction by mutations at the sites I253, H310 and H435 also lead to decreased *in vivo* half-life (Medesan et al., J. Immunol. 158(5): 2211-2217, 1997).

It has been proven that mutations of some residues in the human Fcγ domain that are important for binding to FcRn increase serum half-life. In particular, in human Fcγ1, when three residues are substituted with the other 19 conventional amino acids, some point mutants showed increased FcRn binding affinity (Hinton et al., J. Biol. Chem. 279(8): 6213-6216, 2004). The amino acids of Fc, H310 and H435, and L309 and I253 are known to be mainly involved in the binding to FcRn through a salt bridge and a hydrophobic bond. It was also reported that mutations in T250, M252, S254, T256, V308, E380, M428 and N434 increased or decreased the FcRn-binding affinity (Roopenian et al., Nat. Rview Immunology 7: 715-725, 2007).

Korean Patent No. 10-1027427 discloses Trastuzumab (Herceptin, Genentech) variants having an increased FcRn-binding affinity, and these variants contain one or more amino acid modifications selected from 257C, 257M, 257L, 257N, 257Y, 279Q, 279Y, 308F and 308Y. Korean Patent Publication No. 2010-0099179 provides beacizumab (Avastin, Genentech) variants and these variants show increased *in vivo* half-life by containing amino acid modifications at N434S, M252Y/M428L, M252Y/N434S and M428L/N434S.

The administration of an antibody protein as a therapeutic requires injections with a prescribed frequency in consideration of the clearance and *in vivo* half-life properties of the protein. Longer *in vivo* half-lives allow less frequent injections or a lower dosage, either of which is clearly advantageous. Although the mutations previously reported in the Fc domain yielded some antibody variants with increased FcRn binding affinity and prolonged *in vivo* half-lives, these mutations were found to not be optimal, and some variants did not enhance *in vivo* half-life to a satisfactory level.

Meanwhile, the need to increase *in vivo* half-life is not a problem limited to antibody proteins. Therapeutic proteins, including low molecular weight polypeptides, cytokines and hormones, are easily denatured due to low stability, degraded by proteolytic enzymes in the blood, and finally removed by the action of the kidney or liver. Therefore, protein drugs including polypeptides as pharmaceutically active ingredients should be frequently administered to patients in order to maintain their optimal blood concentration and titer. However, since most protein drugs are administered to patients in injectable formulations, frequent injections for maintaining optimal blood concentration of the active polypeptide cause tremendous pain.

In order to solve these problems, attachment of polymers such as PEG or fusion of proteins such as albumin has been attempted to increase *in vivo* half-life of a polypeptide drug. However, even though PEG is attached or albumin is fused thereto, the polypeptide drug still shows relatively low biological activity or its *in vivo* half-life cannot be increased to a sufficient level. Korean Patent No. 10-0567902 discloses a conjugate that is prepared by linking an immunoglobulin fragment with a non-peptidyl polymer in vitro. In this method, only the immunoglobulin fragment is produced in *E. coli* and then linked to a physiologically active polypeptide through the non-peptidyl polymer, which results in extending the half-life of the polypeptide by minimizing the activity reduction thereof. This method has been recognized as a general technique that can be applied to non-native or synthetic physiologically active substances that are not found in nature as well as to native peptides and proteins. However, there is still a need to maximize the *in vivo* half-life of therapeutic physiologically active substances including peptides and proteins.

Accordingly, the present authors have developed immunoglobulin Fc variants having an increased binding affinity for FcRn, compared to native immunoglobulin Fc fragments. They also found that a protein conjugate in which the immunoglobulin Fc variant of the present disclosure is covalently linked to a physiologically active polypeptide via a non-peptidyl polymer shows more prolonged *in vivo* half-life due to the increased binding affinity for FcRn.

WO 2006/130834 A2 relates to "IgG1 molecules with improved characteristics" such as "improved placental transfer, improved serum half-life, and improved FcRn binding".

### [Disclosure]

### [Technical Problem]

Disclosed herein are immunoglobulin Fc variants having an increased binding affinity for FcRn.

Also disclosed herein are protein conjugates comprising the immunoglobulin Fc variants.

Also disclosed herein is a method for increasing *in vivo* half-life of a physiologically active polypeptide using the immunoglobulin Fc variant.

### [Technical Solution]

The present invention is as defined in the claims. This and other facets of the present disclosure may be more fully understood by reference to the following description.

Disclosed herein but not explicitly claimed is an immunoglobulin Fc variant having an increased binding affinity for FcRn, comprising one or more amino acid modifications selected from the group consisting of 307S, 308F, 380S, 380A, 428L, 429K, 430S, 433K and 434S (this numbering is according to the EU index) in the constant region of a native immunoglobulin Fc fragment.

Also disclosed is a protein conjugate having increased *in vivo* half-life, in which a physiologically active polypeptide is covalently linked to the immunoglobulin Fc variant via a non-peptidyl polymer.

Also disclosed is a method for increasing *in vivo* half-life of a physiologically active polypeptide, comprising the step of covalently linking the immunoglobulin Fc variant to the physiologically active polypeptide via a non-peptidyl polymer.

### [Advantageous Effects]

Since the immunoglobulin Fc variants disclosed herein show a high binding affinity for FcRn, they can increase *in vivo* half-life of a physiologically active polypeptide. Therefore, the protein conjugate having a prolonged *in vivo* half-life, in which the immunoglobulin Fc variant is covalently linked to the physiologically active polypeptide via a non-peptidyl polymer, can be effectively used for the preparation of a long-acting formulation of protein drugs with remarkably low administration frequency.

### [Description of Drawings]

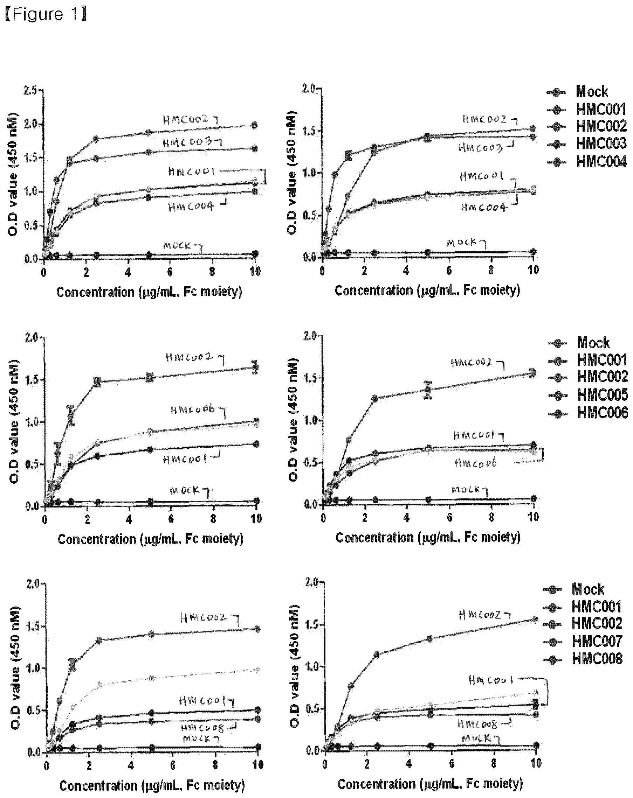

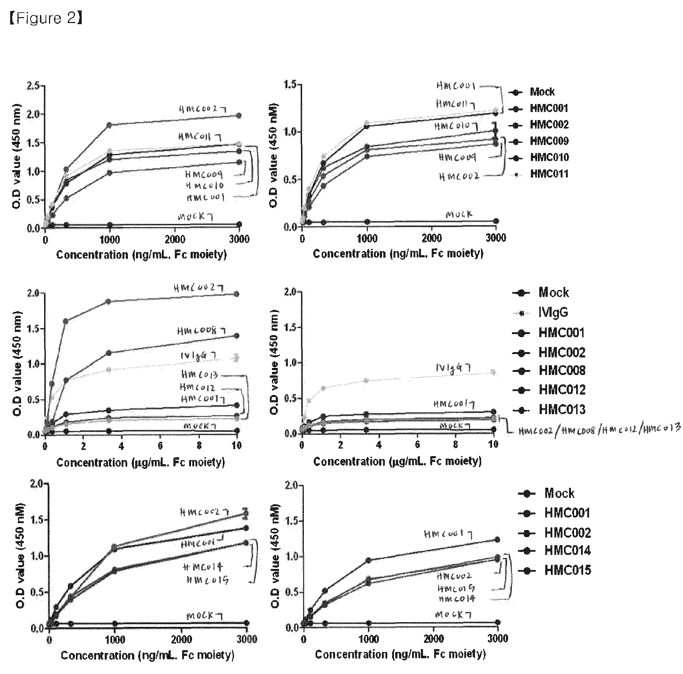

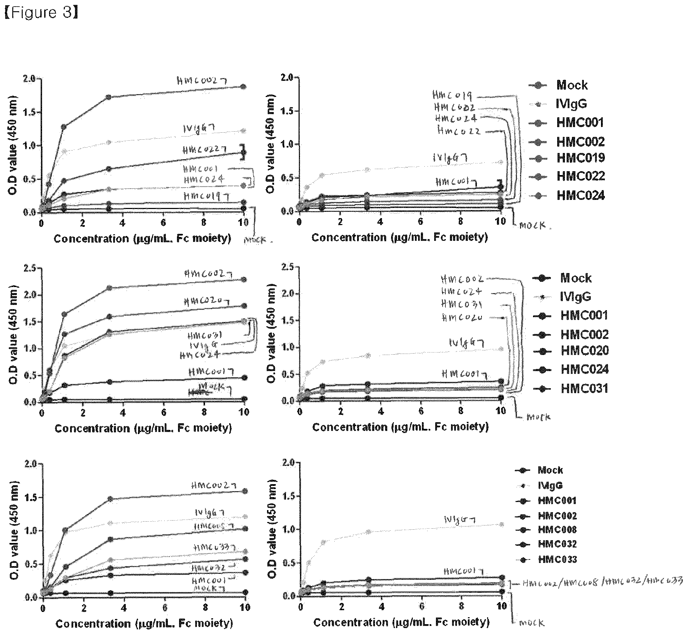

FIGs. 1 to 3 are the results of ELISA for analyzing FcRn-binding affinities of the immunoglobulin Fc variants as disclosed herein, in which the left graphs represent binding affinity at pH 6.0, and the right graphs represent binding affinity at pH 7.4; and

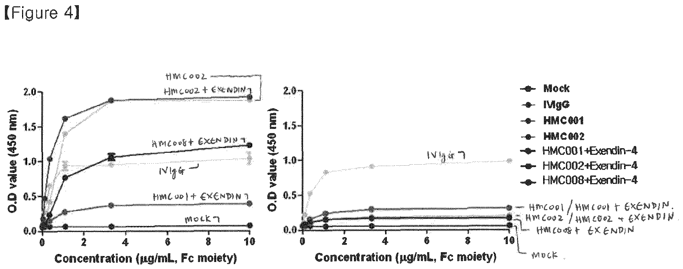

FIG. 4 are the results of ELISA for analyzing FcRn-binding affinities of the protein conjugates disclosed herein, in which the protein conjugate was prepared by linking the immunoglobulin Fc variant and a physiologically active polypeptide via a non-peptidyl polymer, the left graphs represent binding affinity at pH 6.0, and the right graphs represent binding affinity at pH 7.4.

### [Best Mode]

Disclosed herein but not explicitly claimed are immunoglobulin Fc variants having an increased binding affinity for FcRn, which includes one or more amino acid modifications selected from the group consisting of 307S, 308F, 380S, 380A, 428L, 429K, 430S, 433K and 434S (this numbering is according to the EU index) in the constant region of a native immunoglobulin Fc fragment.

As used herein, the term "FcRn" or "neonatal Fc receptor" means a protein that binds to the IgG antibody Fc region and is encoded at least in part by an FcRn gene. The FcRn may be from any organism including humans, mice, rats, rabbits, and monkeys, but is not limited thereto. As is known in the art, the functional FcRn protein includes two polypeptides, often referred to as the heavy chain and the light chain. The light chain is beta-2-microglobulin and the heavy chain is encoded by the FcRn gene. Unless otherwise indicated herein, FcRn or an FcRn protein refers to the complex of FcRn heavy chain with beta-2-microglobulin.

As used herein, the term "native (wild-type) polypeptide" means a non-modified polypeptide that is subjected to modification to generate a variant. The native polypeptide is a naturally occurring polypeptide or a derivative or a manipulated one thereof. The native polypeptide may refer to the polypeptide as it is, a composition including the same, or an amino acid sequence encoding the same. Therefore, the term "native immunoglobulin", as used herein, means a non-modified immunoglobulin polypeptide which generates a variant through amino acid modifications. Interchangeably, the term "parent immunoglobulin", which means a non-modified immunoglobulin polypeptide generating a variant through amino acid modifications, may also be used.

As used herein, the term "amino acid modification" means amino acid substitution, insertion, and/or deletion, preferably substitution in an amino acid sequence. As used herein, the term "amino acid substitution" or "substitution" means the substitution of an amino acid at a particular position in a native polypeptide sequence with another amino acid. For example, an immunoglobulin Fc variant including T307S substitution refers to a variant, in which threonine at position 307 in the amino acid sequence of the native immunoglobulin Fc fragment is substituted with serine.

As used herein, the term "immunoglobulin Fc variant" means to include one or more amino acid modifications, as compared to those of the native immunoglobulin Fc fragment. Preferably, immunoglobulin Fc variant means a variant which includes one or more amino acid modifications selected from the group consisting of 307S, 308F, 380S, 380A, 428L, 429K, 430S, 433K and 434S (this numbering is according to the EU index) so as to have an increased binding affinity for FcRn, as compared to the native immunoglobulin Fc fragment.

The present disclosure is based on the identification of some mutations in the constant region of the immunoglobulin Fc fragment, which show improved binding affinity for FcRn. Disclosed herein are immunoglobulin Fc variants having an increased binding affinity for FcRn and/or *in vivo* half-life, as compared to the corresponding native immunoglobulin Fc fragment. The *in vivo* half-life of an antibody and other physiologically active substances (that is, persistence in the serum or other tissues of a subject) is an important clinical parameter that determines administration dosage and frequency thereof. Therefore, physiologically active substances, including antibodies having a prolonged *in vivo* half-life, are pharmaceutically important and advantageous.

In this regard, there is a report that the *in vivo* half-life of an immunoglobulin correlates with the binding of Fc to FcRn. The immunoglobulin Fc fragment is internalized in acidic endosomes after uptake into endothelial cells via non-specific pinocytosis. FcRn binds to the immunoglobulin at the acidic pH (< 6.5) of endosomes and releases the same at the basic pH (> 7.4) of the bloodstream. Thus, FcRn salvages the immunoglobulin from lysosomal degradation. When a serum immunoglobulin level decreases, more FcRn molecules are available for immunoglobulin binding so that an increased amount of immunoglobulin is salvaged. Conversely, if a serum immunoglobulin level rises, FcRn becomes saturated, thereby increasing the proportion of immunoglobulin that is internalized and degraded (Ghetie and Ward, Annu. Rev. Immunol. 18: 739-766, 2000).

Based on the close relationship between the binding of immunoglobulin Fc fragment to FcRn and *in vivo* half-life of immunoglobulin, a plurality of mutations are introduced into the constant region of a native immunoglobulin Fc fragment in order to develop immunoglobulin Fc variants that show increased binding affinity for FcRn in a low pH environment but substantially no change in binding affinity in a high pH environment. Consequently, modifying one or more amino acids selected from the group consisting of amino acid residues 307, 308, 380, 428, 429, 430, 433 and 434 (this numbering is according to the EU index) in the constant region of the native immunoglobulin Fc fragment has been found to increase the binding affinity for FcRn.

Accordingly, disclosed herein are immunoglobulin Fc variants including one or more amino acid modifications selected from the group consisting of 307S, 308F, 380S, 380A, 428L, 429K, 430S, 433K and 434S (this numbering is according to the EU index) in the constant region of the native immunoglobulin Fc fragment.

The immunoglobulin Fc variants preferably include the amino acid modification selected from the group consisting of 428L/434S, 433K/434S, 429K/433K, 428L/433K, 308F/380A, 307S/380S and 380S/434S in the constant region of the native immunoglobulin Fc fragment.

A specifically disclosed immunoglobulin Fc variant is one in which methionine at position 428 is substituted with leucine and asparagine at position 434 is substituted with serine in the constant region of the native immunoglobulin Fc fragment. The immunoglobulin Fc variant including the amino acid modification of 428L/434S has an amino acid sequence represented by SEQ ID NO: 74, and is encoded by a nucleotide having a base sequence represented by SEQ ID NO: 113. In this disclosure, the immunoglobulin Fc variant is designated as HMC002.

Another specifically disclosed immunoglobulin Fc variant is one in which histidine at position 433 is substituted with lysine and asparagine at position 434 is substituted with serine in the constant region of the native immunoglobulin Fc fragment. The immunoglobulin Fc variant including the amino acid modification of 433K/434S has an amino acid sequence represented by SEQ ID NO: 80, and is encoded by a nucleotide having a base sequence represented by SEQ ID NO: 114. In this disclosure, the immunoglobulin Fc variant is designated as HMC008. In the claimed protein conjugates of the invention the immunoglobulin Fc variant has an amino acid sequence represented by SEQ ID NO: 80.

Another specifically disclosed immunoglobulin Fc variant is one in which histidine at position 429 is substituted with lysine and histidine at position 433 is substituted with lysine in the constant region of the native immunoglobulin Fc fragment. The immunoglobulin Fc variant including the amino acid modification of 429K/433K has an amino acid sequence represented by SEQ ID NO: 91, and is encoded by a nucleotide having a base sequence represented by SEQ ID NO: 115. In this disclosure, the immunoglobulin Fc variant is designated as HMC019.

Another specifically disclosed immunoglobulin Fc variant is one in which methionine at position 428 is substituted with leucine and histidine at position 433 is substituted with lysine in the constant region of the native immunoglobulin Fc fragment. The immunoglobulin Fc variant, including the amino acid modification of 428L/433K, has an amino acid sequence represented by SEQ ID NO: 92 and is encoded by a nucleotide having a base sequence represented by SEQ ID NO: 116. In this diclsosure, the immunoglobulin Fc variant is designated as HMC020.

Another specifically disclosed immunoglobulin Fc variant is one in which valine at position 308 is substituted with phenylalanine and glutamic acid at position 380 is substituted with alanine in the constant region of the native immunoglobulin Fc fragment. The immunoglobulin Fc variant, including the amino acid modification of 308F/380A, has an amino acid sequence represented by SEQ ID NO: 100 and is encoded by a nucleotide having a base sequence represented by SEQ ID NO: 117. In this disclosure, the immunoglobulin Fc variant is designated as HMC028.

Another specifically disclosed immunoglobulin Fc variant is one in which threonine at position 307 is substituted with serine and glutamic acid at position 380 is substituted with serine in the constant region of the native immunoglobulin Fc fragment. The immunoglobulin Fc variant, including the amino acid modification of 307S/380S, has an amino acid sequence represented by SEQ ID NO: 101, and is encoded by a nucleotide having a base sequence represented by SEQ ID NO: 118. In this disclosure, the immunoglobulin Fc variant is designated as HMC029.

Another specifically disclosed immunoglobulin Fc variant is one in which glutamic acid at position 380 is substituted with serine and asparagine at position 434 is substituted with serine in the constant region of the native immunoglobulin Fc fragment. The immunoglobulin Fc variant including the amino acid modification of 380S/434S has an amino acid sequence represented by SEQ ID NO: 103 and is encoded by a nucleotide having a base sequence represented by SEQ ID NO: 119. In this disclosure, the immunoglobulin Fc variant is designated as HMC031.

The parent immunoglobulin Fc fragment to be used for the preparation of the above described immunoglobulin Fc variants may be preferably HMC001 produced from an *E*. *coli* transformant HM11201 (KCCM-10660P) that is disclosed in Korean Patent No. 10-824505. HMC001 is an immunoglobulin Fc fragment having an amino acid sequence represented by SEQ ID NO: 73.

In this disclosure the immunoglobulin Fc variants are defined according to the amino acid modifications introduced into the parent immunoglobulin Fc fragment and the numbering of the amino acid residues therein is that of the EU index as in Kabat (Kabat et al., Sequence of proteins of immunological interest, 5th Ed., United States Public Health Service, National Institutes of Health, Bethesda, 1991). At this time, since HMC001 used as the parent
immunoglobulin Fc fragment is an immunoglobulin Fc fragment that is devoid of an initial methionine residue by removal of a part of the N-terminus upon production in the *E. coli* transformant, it may not be according to the Kabat numbering. HMC001 used as the parent immunoglobulin Fc fragment has proline as a first amino acid, and the amino acid numbering of the immunoglobulin Fc variants disclosed herein complies therewith. However, the mutation positions introduced into the immunoglobulin Fc variants are not limited to HMC001 and may be defined according to the Kabat numbering. For example, the "81T" of HCM001 is the same as the "307T" according to the Kabat numbering.

The immunoglobulin Fc variants disclosed herein show increased binding affinity at low pH, for example, at pH 6.0 of endosomes, but no increased binding affinity at high pH, for example, at pH 7.4. Further, their internalization into endosomes increases, but the immunoglobulin Fc variants can be released at a normal rate, resulting in increased *in vivo* half-life.

In this disclosure, the native immunoglobulin Fc fragment may be an Fc fragment derived from human IgG1, IgG2, IgG3 or IgG4. In this disclosure, the native immunoglobulin Fc fragment may be preferably an Fc fragment derived from human IgG4, which does not include the variable region and the heavy chain, and is aglycosylated.

The immunoglobulin Fc variants include one or more amino acid modifications, as compared to the native immunoglobulin Fc fragment, and therefore have different amino acid sequences. The amino acid sequences of the immunoglobulin Fc variants are substantially homologous to that of the native immunoglobulin Fc fragment. For example, the amino acid sequences of the immunoglobulin Fc variants may have approximately 80% or higher homology, preferably approximately 90% or higher homology, and most preferably approximately 95% or higher homology than that of the native immunoglobulin Fc fragment. The amino acid modification may be genetically performed by a molecular biological method or may be performed by an enzymatic or chemical method.

The immunoglobulin Fc variants may be prepared by any conventional method known in the art. In some cases, the immunoglobulin Fc variants are used to create nucleic acids that encode the polypeptide sequences including particular amino acid modifications, followed by being cloned into host cells, expressed and assayed, if desired. A variety of methods are described in relevant literature (Molecular Cloning - A Laboratory Manual, 3rd Ed., Maniatis, Cold Spring Harbor Laboratory Press, New York, 2001; Current Protocols in Molecular Biology, John Wiley & Sons).

The nucleic acids that encode the immunoglobulin Fc variants may be incorporated into an expression vector for protein expression. Expression vectors typically include a protein operably linked, that is, placed in a functional relationship, with control or regulatory sequences, selectable markers, any fusion partners, and/or additional elements. The immunoglobulin Fc variant may be produced by culturing a host cell transformed with the nucleic acid, preferably an expression vector containing the nucleic acid encoding the immunoglobulin Fc variant, under conditions appropriate so as to induce or cause the expression thereof. A wide variety of appropriate host cells include, but are not limited to, mammalian cells, bacterial cells, insect cells, and yeast cells. The methods of introducing an exogenous nucleic acid into host cells are well known in the art, and will vary with the host cell used. *E. coli,* which is industrially valuable due to low production costs, can preferably be used as a host cell to produce the immunoglobulin Fc variants.

Therefore, disclosed herein but not claimed is a method for preparing the immunoglobulin Fc variant, comprising the steps of:
1) culturing the host cells, into which the nucleic acid encoding the immunoglobulin Fc variant is introduced, under the conditions suitable for protein expression; and
2) purifying or isolating the immunoglobulin Fc variant expressed from the host cells.

Antibodies may be isolated or purified by various methods known in the art. Standard purification methods include chromatographic techniques, electrophoresis, immunoprecipitation, dialysis, filtration, concentration, and chromatofocusing techniques. As is well known in the art, a variety of natural proteins such as bacterial proteins A, G, and L can bind to antibodies, and thus these proteins can be used for the purification of antibodies. Purification can often be enabled by using a particular fusion partner. For example, proteins may be purified by using a glutathione resin if a GST fusion is employed, by using a Ni⁺² affinity chromatography if a His-tag is employed, or by using an immobilized anti-flag antibody if a flag-tag is used.

The present invention relates to a protein conjugate having an increased *in vivo* half-life, in which a physiologically active polypeptide is covalently linked to an immunoglobulin Fc variant via a non-peptidyl polymer. In the claimed protein conjugates, the non-peptidyl polymer is a polyethylene glycol, the immunoglobulin Fc variant has an amino acid sequence represented by SEQ ID NO: 80, and the physiologically active polypeptide is selected from the group consisting of insulin, glucagon, glucagon like peptide-1 and exendin-4.

The immunoglobulin Fc variants show increased binding affinity at low pH of endosomes, for example, at pH 6.0, whereas no corresponding increased binding affinity at high pH, for example, at pH 7.4. Thus, their internalization into endosomes increases, but they are released at a normal rate, leading to increased *in vivo* half-life (see FIG. 1). Therefore, the immunoglobulin Fc variants disclosed herein can be used as a carrier for increasing *in vivo* half-life of physiologically active polypeptides including protein drugs. Accordingly, the protein conjugate disclosed herein can be effectively used in the preparation of a long-acting drug formulation having remarkably increased *in vivo* half-life due to high binding affinity for FcRn.

Further, the present invention provides a method for preparing a long-acting drug formulation by covalently linking the immunoglobulin Fc variant of the present invention to a physiologically active polypeptide via a non-peptidyl polymer. In the claimed methods, the non-peptidyl polymer is a polyethylene glycol, the immunoglobulin Fc variant has an amino acid sequence represented by SEQ ID NO: 80, and the physiologically active polypeptide is selected from the group consisting of insulin, glucagon, glucagon like peptide-1 and exendin-4.

The preparation method disclosed herein may comprise the steps of:
1) covalently linking the immunoglobulin Fc variant to the physiologically active polypeptide via the non-peptidyl polymer having a terminal reactive group; and
2) isolating a conjugate in which the physiologically active polypeptide, the non-peptidyl polymer, and the immunoglobulin Fc variant are covalently linked to each other.

Disclosed non-peptidyl polymers which do not necessarily form part of the claimed subject matter include the group consisting of biodegradable polymers such as polyethylene glycol, polypropylene glycol, a copolymer of ethylene glycol and propylene glycol, polyoxyethylated polyol, polyvinyl alcohol, polysaccharide, dextran, polyvinyl ethyl ether, PLA (polylactic acid) and PLGA (polylactic-glycolic acid), lipopolymers, chitins, hyaluronic acid and combinations thereof, and preferably polyethylene glycol. Also, their derivatives that are known in the art or that can be readily prepared using a conventional technique fall within the scope of the present disclosure.

Disclosed physiologically active polypeptide to be linked to the immunoglobulin Fc variant, and which do not necessarily form part of the claimed subject matter, may be any one without limitation, as long as it is needed to have increased *in vivo* half-life. For example, various physiologically active polypeptides that are used for the purpose of treating or preventing human diseases, such as cytokines, interleukins, interleukin-binding proteins, enzymes, antibodies, growth factors, transcription factors, blood factors, vaccines, structural proteins, ligand proteins or receptors, cell surface antigens, and receptor antagonists, and derivatives or analogs thereof.

Examples of the physiologically active polypeptides which do not necessarily form part of the claimed subject matter include human growth hormones, growth hormone releasing hormones, growth hormone releasing peptides, interferons and interferon receptors (e.g., interferon-alpha, -beta and -gamma, soluble type I interferon receptors), granulocyte colony-stimulating factors (G-CSF), granulocyte-macrophage colony-stimulating factors (GM-CSF), glucagon-like peptides (GLP-1), G-protein-coupled receptors, interleukins (e.g., IL-1 receptors, IL-4 receptors), enzymes (e.g., glucocerebrosidase, iduronate-2-sulfatase, alpha-galactosidase-A, agalsidase alpha, beta, alpha-L-iduronidase, butyrylcholinesterase, chitinase, glutamate decarboxylase, imiglucerase, lipase, uricase, platelet-activatingfactor acetylhydrolase, neutral endopeptidase, myeloperoxidase), interleukin- and cytokine-binding proteins (e.g., IL-18 bp, TNF-binding protein), macrophage activating factors, macrophage peptides, B-cell factors, T-cell factors, Protein A, allergy inhibitors, cell necrosis glycoproteins, immunotoxins, lymphotoxins, tumor necrosis factor, tumor suppressors, transforming growth factor, alpha-1 anti-trypsin, albumin, alpha-lactalbumin, apolipoprotein-E, erythropoietin, highly glycosylated erythropoietin, angiopoietins, hemoglobin, thrombin, thrombin receptors activating peptides, thrombomodulin, blood factor VII, blood factor VIIa, blood factor VIII, blood factor IX and blood factor XIII, plasminogen activators, fibrin-binding peptides, urokinase, streptokinase, hirudin, Protein C, C-reactive protein, renin inhibitor, collagenase inhibitor, superoxide dismutase, leptin, platelet-derived growth factor, epithelial growth factor, epidermal growth factor, angiostatin, endostatin angiotensin, bone growth factor, bone stimulating protein, calcitonin, insulin, atriopeptin, cartilage inducing factor, elcatonin, connective tissue activating factor, tissue factor pathway inhibitor, follicle stimulating hormone, luteinizing hormone, luteinizing hormone releasing hormone, nerve growth factors (e.g., nerve growth factor, cilliary neurotrophic factor, axogenesis factor-1, brain-natriuretic peptide, glial derived neurotrophic factor, netrin, neurophil inhibitory factor, neurotrophic factor, neuturin), parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, autotaxin, lactoferrin, myostatin, receptors (e.g., TNFR(P75), TNFR(P55), IL-1 receptor, VEGF receptor, B-cell activator receptor), receptor antagonists (e.g., IL1-Ra), cell surface antigens (e.g., CD 2, 3, 4, 5, 7, 11a, 11b, 18, 19, 20, 23, 25, 33, 38, 40, 45, 69), monoclonal antibodies, polyclonal antibodies, antibody fragments (e.g., scFv, Fab, Fab', F(ab')2 and Fd), and virus derived vaccine antigens, but are not limited thereto. The antibody fragments may be selected from Fab, Fab', F(ab')₂, Fd and scFv having an ability to bind to a particular antigen.

The protein conjugate in which the physiologically active polypeptide is covalently linking to the immunoglobulin Fc variant via the non-peptidyl polymer shows remarkably prolonged *in vivo* half-life due to a high binding affinity for FcRn (see FIG. 4). Therefore, the protein conjugate that is prepared by using the immunoglobulin Fc fragment as a carrier can increase persistence in the blood, and thereby remarkably reduce administration frequency.

The present disclosure is further illustrated by the following Examples.

### Example 1: Construction of a vector expressing an immunoglobulin Fc fragment

The positions involved in binding affinity for human FcRn were selected from the amino acid sequence (SEQ ID NO: 73) of HMC001 produced from the *E. coli* transformant HM11201 (KCCM-10660P, Korean Patent No. 10-0824505), and mutagenesis was induced thereon to substitute the amino acid of the corresponding position with another amino acid so as to increase the binding affinity for FcRn. To achieve this, primers for nucleotide substitution and a QuikChange^{™} Site-directed Mutagenesis kit (Stratagene) were used. In this regard, the primers used in site-directed mutagenesis are shown in the following Tables 1 to 3.

For site-directed mutagenesis by polymerase chain reaction (PCR), 50 ng of an HMC001 expression vector, each primer pair, dNTP and PfuTurbo^{™} polymerase (Stratagene) were added in a PCR tube, and reaction was performed as follows: denaturation at 95°C for 30 seconds, 16 cycles of 95°C for 30 seconds, 55°C for 1 minute and 68°C for 14 minutes, and final extension of 68°C 5 minutes. PCR products of approximately 1.2 kb amplified above were subjected to base sequence analysis through DNA sequencing, and the results are shown in the following Table 4. The amino acid numbering described in Table 4 is according to the EU index, and the number in parentheses represents the amino acid numbering based on the amino acid sequence of HMC001 (SEQ ID NO: 73).

Each of the amplified PCR products was cleaved with Ndel and BamHI and then inserted into a plasmid pET22b (Novagen) that was previously treated with the same restriction enzymes to thereby obtain expression vectors including each of the immunoglobulin Fc variants.

**[Table 1]**

| Variant | Primer | Base sequence | SEQ ID NO |
|---|---|---|---|
| HMC002 | FcM202Lss | CTTCTCATGCTCCGTGCTGCATGAGGCTCT GCAC | 1 |
| HMC023 | | | |
| HMC002 | FcM202Las | GTGCAGAGCCTCATGCAGCACGGAGCATG AGAAG | 2 |
| HMC023 | | | |
| HMC002 | FcN208Sss | CATGAGGCTCTGCACAGCCACTACACACA GAAG | 3 |
| HMC006 | | | |
| HMC026 | | | |
| HMC031 | | | |
| HMC036 | | | |
| HMC037 | | | |
| HMC038 | | | |
| HMC040 | | | |
| HMC002 | FcN208Sas | CTTCTGTGTGTAGTGGCTGTGCAGAGCCTC ATG | 4 |
| HMC006 | | | |
| HMC026 | | | |
| HMC031 | | | |
| HMC036 | | | |
| HMC037 | | | |
| HMC038 | | | |
| HMC040 | | | |
| HMC003 | FcI27Fss | CAAGGACACCCTCATGTTCTCCCGGACCCC TGAG | 5 |
| HMC003 | FcI27Fas | CTCAGGGGTCCGGGAGAACATGAGGGTGT | 6 |
| | | CCTTG | |
| HMC004 | FcH207Tss | GATGCATGAGGCTCTGACCAACCACTACA CACAG | 7 |
| HMC004 | FcH207Tas | CTGTGTGTAGTGGTTGGTCAGAGCCTCATG CATC | 8 |
| HMC005 | FcH207Kss | GATGCATGAGGCTCTGAAGAACCACTACA CACAG | 9 |
| HMC005 | FcH207Kas | CTGTGTGTAGTGGTTCTTCAGAGCCTCATGC ATC | 10 |
| HMC006 | FcL83Fss | CAGCGTCCTCACCGTCTTCCACCAGGACTG GCTG | 11 |
| HMC011 | | | |
| HMC006 | FcL83Fas | CAGCCAGTCCTGGTGGAAGACGGTGAGGA CGCTG | 12 |
| HMC011 | | | |
| HMC007 | FcI27Lss | CAAGGACACCCTCATGCTCTCCCGGACCCC TGAG | 13 |
| HMC007 | FcI27Las | CTCAGGGGTCCGGGAGAGCATGAGGGTGT CCTTG | 14 |
| HMC008 | FcH207K/N208Sss | GATGCATGAGGCTCTGAAGAGCCACTACA | 15 |
| | | CACAG | |
| HMC008 | FcH207K/N208Sas | CTGTGTGTAGTGGCTCTTCAGAGCCTCATG CATC | 16 |
| HMC009 | FcN208Tss | CATGAGGCTCTGCACACCCACTACACACA GAAG | 17 |
| HMC009 | FcN208Tas | CTTCTGTGTGTAGTGGGTGTGCAGAGCCTC ATG | 18 |
| HMC010 | FcH207T/N208Sss | GATGCATGAGGCTCTGACCAGCCACTACA CACAG | 19 |
| HMC010 | FcH207T/N208Sas | CTGTGTGTAGTGGCTGGTCAGAGCCTCATG CATC | 20 |
| HMC012 | FcE204Rss | ATGCTCCGTGATGCATCGGGCTCTGCACAA CCAC | 21 |
| HMC012 | FcE204Ras | GTGGTTGTGCAGAGCCCGATGCATCACGG AGCAT | 22 |
| HMC013 | FcE204Kss | ATGCTCCGTGATGCATAAGGCTCTGCACAA CCAC | 23 |
| HMC013 | FcE204Kas | GTGGTTGTGCAGAGCCTTATGCATCACGGA | 24 |
| | | GCAT | |

**[Table 2]**

| Variant | Primer | Base sequence | SEQ ID NO |
|---|---|---|---|
| HMC014 | FcE204R/H207Kss | TGCTCCGTGATGCATCGGGCTCTGAAGAAC CACTACACACAG | 25 |
| HMC014 | FcE204R/H207Kas | CTGTGTGTAGTGGTTCTTCAGAGCCCGATG CATCACGGAGCA | 26 |
| HMC015 | FcE204K/H207Kss | TGCTCCGTGATGCATAAGGCTCTGAAGAAC CACTACACACAG | 27 |
| HMC015 | FcE204K/H207Kas | CTGTGTGTAGTGGTTCTTCAGAGCCTTATG CATCACGGAGCA | 28 |
| HMC016 | FcH203Rss | CTCATGCTCCGTGATGCGTGAGGCTCTGCA CAAC | 29 |
| HMC016 | FcH203Ras | GTTGTGCAGAGCCTCACGCATCACGGAGC ATGAG | 30 |
| HMC017 | FcH203Kss | CTCATGCTCCGTGATGAAGGAGGCTCTGC | 31 |
| | | ACAAC | |
| HMC017 | FcH203Kas | GTTGTGCAGAGCCTCCTTCATCACGGAGC ATGAG | 32 |
| HMC018 | FcH203R/H207Kss | CATGCTCCGTGATGCGTGAGGCTCTGAAG AACCACTACACACAG | 33 |
| HMC018 | FcH203R/H207Kas | CTGTGTGTAGTGGTTCTTCAGAGCCTCACG CATCACGGAGCATG | 34 |
| HMC019 | FcH203K/H207Kss | CATGCTCCGTGATGAAGGAGGCTCTGAAG AACCACTACACACAG | 35 |
| HMC019 | FcH203K/H207Kas | CTGTGTGTAGTGGTTCTTCAGAGCCTCCTTC ATCACGGAGCATG | 36 |
| HMC020 | FcM202L/H207Kss | CTTCTCATGCTCCGTGCTGCATGAGGCTCT GAAGAACCACTACACACAC | 37 |
| HMC020 | FcM202L/H207Kas | CTGTGTGTAGTGGTTCTTCAGAGCCTCATG CAGCACGGAGCATGAGAAG | 38 |
| HMC021 | FcH203K/N208Tss | CTCATGCTCCGTGATGAAGGAGGCTCTGC ACACCCACTACACACAGAAG | 39 |
| HMC021 | FcH203K/N208Tas | CTTCTGTGTGTAGTGGGTGTGCAGAGCCTC | 40 |
| | | CTTCATCACGGAGCATGAG | |
| HMC022 | FcT81Ess | GTGGTCAGCGTCCTCGAAGTCCTGCACCA GGAC | 41 |
| HMC023 | | | |
| HMC022 | FcT81 Eas | GTCCTGGTGCAGGACTTCGAGGACGCTGA CCAC | 42 |
| HMC023 | | | |
| HMC024 | FcE154Ass | AGCGACATCGCCGTGGCGTGGGAGAGCA ATGGG | 43 |
| HMC026 | | | |
| HMC028 | | | |
| HMC024 | FcE154Aas | CCCATTGCTCTCCCACGCCACGGCGATGTC GCT | 44 |
| HMC026 | | | |
| HMC028 | | | |

**[Table 3]**

| Variant | Primer | Base sequence | SEQ ID NO |
|---|---|---|---|
| HMC025 | FcE154Sss | AGCGACATCGCCGTGTCGTGGGAGAGCAA TGGG | 45 |
| HMC029 | | | |
| HMC031 | | | |
| HMC025 | FcE154Sas | CCCATTGCTCTCCCACGACACGGCGATGTC GCT | 46 |
| HMC029 | | | |
| HMC031 | | | |
| HMC027 | FcV82Pss | GTCAGCGTCCTCACCCCCCTGCACCAGGA CTGG | 47 |
| HMC028 | | | |
| HMC027 | FcV82Pas | CCAGTCCTGGTGCAGGGGGGTGAGGACG CTGAC | 48 |
| HMC028 | | | |
| HMC027 | FcE204Sss | TGCTCCGTGATGCATTCGGCTCTGCACAAC CAC | 49 |
| HMC027 | FcE204Sas | GTGGTTGTGCAGAGCCGAATGCATCACGG AGCA | 50 |
| HMC029 | FcT81Sss | GTGGTCAGCGTCCTCTCCGTCCTGCACCAG GAC | 51 |
| HMC029 | FcT81Sas | GTCCTGGTGCAGGACGGAGAGGACGCTG ACCAC | 52 |
| HMC030 | FcH203A/E204Sss | TCATGCTCCGTGATGGCTTCGGCTCTGCAC AACC | 53 |
| HMC030 | FcH203A/E204Sas | GGTTGTGCAGAGCCGAAGCCATCACGGAG | 54 |
| | | CATGA | |
| HMC032 | FcH207A/N208Sss | GATGCATGAGGCTCTGGCCAGCCACTACA CACAG | 55 |
| HMC032 | FcH207A/N208Sas | CTGTGTGTAGTGGCTGGCCAGAGCCTCATG CATC | 56 |
| HMC033 | FcE204A/N208Sss | GCTCCGTGATGCATGCGGCTCTGCACAGCC AC | 57 |
| HMC033 | FcE204A/N208Sas | GTGGCTGTGCAGAGCCGCATGCATCACGG AGC | 58 |
| HMC034 | FcH203A/N208Sss | CTCATGCTCCGTGATGGCTGAGGCTCTGCA CAGC | 59 |
| HMC034 | FcH203A/N208Sas | GCTGTGCAGAGCCTCAGCCATCACGGAGC ATGAG | 60 |
| HMC035 | FcM202A/N208Sss | CTTCTCATGCTCCGTGGCGCATGAGGCTCT GCAC | 61 |
| HMC035 | FcM202A/N208Sas | GTGCAGAGCCTCATGCGCCACGGAGCATG AGAAG | 62 |
| HMC036 | FcY93 Lss | GCTGAATGGCAAGGAGCTCAAGTGCAAG | 63 |
| | | GTCTCC | |
| HMC036 | FcY93Las | GGAGACCTTGCACTTGAGCTCCTTGCCATT CAGC | 64 |
| HMC037 | FcV82Lss | GGTCAGCGTCCTCACCCTCCTGCACCAGG ACTG | 65 |
| HMC037 | FcV82Las | CAGTCCTGGTGCAGGAGGGTGAGGACGCT GACC | 66 |
| HMC038 | FcT81Ass | GTGTGGTCAGCGTCCTCGCCGTCCTGCACC AGGA | 67 |
| HMC038 | FcT81Aas | TCCTGGTGCAGGACGGCGAGGACGCTGAC CACAC | 68 |
| HMC039 | FcH207L/N208Sss | GATGCATGAGGCTCTGCTCAGCCACTACAC ACAG | 69 |
| HMC039 | FcH207L/N208Sas | CTGTGTGTAGTGGCTGAGCAGAGCCTCATG CATC | 70 |
| HMC040 | FcY93A/N208Sss | GCTGAATGGCAAGGAGGCCAAGTGCAAG GTCTCC | 71 |
| HMC040 | FcY93A/N208Sa | GGAGACCTTGCACTTGGCCTCCTTGCCATT | 72 |
| | | CAGC | |

**[Table 4]**

| Modif icatio n positi on* | 253 | 307 | 308 | 309 | 319 | 380 | 428 | 429 | 430 | 433 | 434 | SEQ ID NO |
|---|---|---|---|---|---|---|---|---|---|---|---|---|
| | (27) | (81) | (82) | (83) | (93) | (154) | (202) | (203) | (204) | (207) | (208) | |
| Modif ied AA | Ile | Thr | Val | Leu | Tyr | Glu | Met | His | Glu | His | Asn | |
| HMC 002 | | | | | | | Leu | | | | Ser | 74 |
| HMC 003 | Phe | | | | | | | | | | | 75 |
| HMC | | | | | | | | | | Thr | | 76 |
| 004 | | | | | | | | | | | | |
| HMC 005 | | | | | | | | | | Lys | | 77 |
| HMC 006 | | | | Phe | | | | | | | Ser | 78 |
| HMC 007 | Leu | | | | | | | | | | | 79 |
| HMC 008 | | | | | | | | | | Lys | Ser | 80 |
| HMC 009 | | | | | | | | | | | Thr | 81 |
| HMC 010 | | | | | | | | | | Thr | Ser | 82 |
| HMC 011 | | | | Phe | | | | | | | | 83 |
| HMC 012 | | | | | | | | | Arg | | | 84 |
| HMC | | | | | | | | | Lys | | | 85 |
| 013 | | | | | | | | | | | | |
| HMC 014 | | | | | | | | | Arg | Lys | | 86 |
| HMC 015 | | | | | | | | | Lys | Lys | | 87 |
| HMC 016 | | | | | | | | Arg | | | | 88 |
| HMC 017 | | | | | | | | Lys | | | | 89 |
| HMC 018 | | | | | | | | Arg | | Lys | | 90 |
| HMC 019 | | | | | | | | Lys | | Lys | | 91 |
| HMC 020 | | | | | | | Leu | | | Lys | | 92 |
| HMC 021 | | | | | | | | Lys | | | Thr | 93 |
| HMC | | Glu | | | | | | | | | | 94 |
| 022 | | | | | | | | | | | | |
| HMC 023 | | Glu | | | | | Leu | | | | | 95 |
| HMC 024 | | | | | | Ala | | | | | | 96 |
| HMC 025 | | | | | | Ser | | | | | | 97 |
| HMC 026 | | | | | | Ala | | | | | Ser | 98 |
| HMC 027 | | | Phe | | | | | | Ser | | | 99 |
| HMC 028 | | | Phe | | | Ala | | | | | | 100 |
| HMC 029 | | Ser | | | | Ser | | | | | | 101 |
| HMC 030 | | | | | | | | Ala | Ser | | | 102 |
| HMC | | | | | | Ser | | | | | Ser | 103 |
| 031 | | | | | | | | | | | | |
| HMC 032 | | | | | | | | | | Ala | Ser | 104 |
| HMC 033 | | | | | | | | | Ala | | Ser | 105 |
| HMC 034 | | | | | | | | Ala | | | Ser | 106 |
| HMC 035 | | | | | | | Ala | | | | Ser | 107 |
| HMC 036 | | | | | Leu | | | | | | Ser | 108 |
| HMC 037 | | | Leu | | | | | | | | Ser | 109 |
| HMC 038 | | Ala | | | | | | | | | Ser | 110 |
| HMC 039 | | | | | | | | | | Leu | Ser | 111 |
| HMC | | | | | | | | | | | Ser | 112 |
| 040 | | | | | | | | | | His | | |
| | | | | | | | | | | | | |

### Example 2: Production of immunoglobulin Fc variants in E. coli

The expression vectors of immunoglobulin Fc variants prepared in Example 1 were transformed into *E. coli* BL21 (DE3) competent cells (Invitrogen) by heat shock at 42°C for 1 minute, respectively, followed by culturing on LB solid media supplemented with ampicillin to select colonies resistant to ampicillin.

Thus selected colonies were inoculated in 500 ml of 2× LB media (containing ampicillin), and cultured in a shaking incubator at 37°C and 120 rpm for 15hours. After 100 ml of the culture solution was transferred to 500 ml of fresh 2× LB media (containing ampicillin), the resulting solution was incubated until the optical density at 600 nm (OD600) reached 4 to 5. 200 ml of the culture solution was transferred to a 5 L-fermentor at a ratio of 10% (v/v), 2× LB media (containing ampicillin) was injected thereto, and a semi-pH stat fed-batch culture was performed under the conditions of 37°C, 500-700 rpm and an aeration rate of 1 vvm for 45 hours. During fermentation, when the OD600 reached 90 or higher, IPTG (isopropyl-1-thio-β-D-galactopyranoside) was added to the fermentor as an inducer at a final concentration of 0.1 mM so as to induce protein synthesis.

### Example 3: Isolation and purification of immunoglobulin Fc variants

The culture solution fermented in Example 2 was centrifuged at 12,000 g for 30 minutes to recover a cell pellet. Thus recovered cell pellet was suspended in 10× volumes of a lysis buffer (20 mM Tris (pH 9.0), 1 mM EDTA (pH 8.0), 0.2 M NaCl, 0.5% triton X-100), and then disrupted three times using a microfluidizer (Microfluidics) at a pressure of 15,000 psi. Thus obtained cell lysate solution was centrifuged at 6,000 g for 30 minutes to obtain only inclusion bodies of the proteins expressed by the *E. coli* transformant.

The inclusion bodies were washed with 0.5% triton X-100 and distilled water and suspended in an 8 M urea solution containing 10× volumes of 20 mM Tris (pH 9.0) for 2 hours to dissolve them. In order to separate insoluble solid impurities, the inclusion body-dissolved solution was centrifuged at 12,000 g for 30 minutes to collect a supernatant. Then, L-cysteine was added to the supernatant at a final concentration of 1 mM and incubated at room temperature for 1 hour to induce protein reduction.

For refolding of the reduced protein, the inclusion bodies dissolved at 4°C for 24 hours were diluted with 100× volumes of a refolding solution (2 M urea, 0.25 M arginine, 50 mM Tris (pH 8.5), 0.5 mM Cys)

Thus refolded proteins were purified in an Akta purifier (Amersham Pharmacia Biotech) using an ion exchange column (IEX column, GE Healthcare) to thereby obtain immunoglobulin Fc variants with a purity of 98% or higher.

### Example 4: Evaluation of human FcRn-binding affinity of immunoglobulin Fc variants

To evaluate FcRn-binding affinity of the immunoglobulin Fc variant isolated and purified in Example 3, ELISA was performed. In the following experiment, human FcRn (hFcRn, human neonatal Fcγ receptor) was to be expressed in 293T cells and purified therefrom, and a GST antibody was purchased from Merk Milipore. The FcRn-binding affinity measured in each of the immunoglobulin Fc variants was compared with those of HMC001 and native IgG as a control group.

After a 96-well plate was coated with 30 µg/ml of native IgG and each 10 µg/ml of HMC001 and the immunoglobulin Fc variant, 100 µl of 5 µg/ml FcRn was added to each well. For evaluation of binding and dissociation, the experiment was performed at pH 6.0 and 7.4. An assay buffer and a washing buffer used in this experiment have the following composition as shown in Table 5, and the results are shown in FIGs. 1 to 3.

**[Table 5]**

| | Composition |
|---|---|
| Assay buffer | Sodium phosphate (pH 6.0/7.4), 0.5% BSA, 0.05% Tween 20 |
| Washing buffer | Sodium phosphate (pH 6.0/7.4), 0.05% Tween 20 |

As shown in FIGs. 1 to 3, the immunoglobulin Fc variants were found to show low FcRn-binding affinity at pH 7.4, but high FcRn-binding affinity at pH 6.0, as compared to HMC001 and native IgG.

### Example 5: Evaluation of human FcRn-binding affinity of a conjugate comprising the immunoglobulin Fc variant and exendin-4

In order to examine whether the immunoglobulin Fc variants show increased binding affinity for human FcRn even in a conjugated form with a protein drug, the immunoglobulin Fc variants, HMC002 and HMC008 that were found to show high FcRn-binding affinity in Example 4 were covalently linked to exendin-4 using PEG having aldehyde reactive groups at both ends, to thereby prepare protein conjugates. Preparation of the protein conjugates was performed in accordance with the method described in Korean Patent No. 10-1058290. ELISA was performed according to the same method as described in Example 4 to evaluate human FcRn-binding affinity of the protein conjugates prepared above.

As shown in FIG. 4, it was found that the immunoglobulin Fc variants maintained high FcRn-binding affinity, even though each of them was linked to a physiologically active polypeptide via a non-peptidyl polymer.

Therefore, the drug conjugate prepared by using the immunoglobulin Fc variant as a carrier has greatly prolonged *in vivo* half-life owing to the increased binding affinity for FcRn, and thus it can be used as a long-acting drug formulation capable of remarkably reducing administration frequency.

Singular forms used in the present description include plural forms unless they apparently represent opposite meanings. The meaning of "including" or "having" used in the present description is intended to embody specific properties, regions, integers, steps, operations, elements and/or components, but is not intended to exclude presence or addition of other properties, regions, integers, steps, operations, elements, components and/or groups.

### [Industrial Applicability]

The immunoglobulin Fc variants disclosed herein show a high binding affinity for FcRn, and thus can increase *in vivo* half-life of a physiologically active polypeptide. Therefore, the protein conjugate having a prolonged *in vivo* half-life, in which the immunoglobulin Fc variant is covalently linked to the physiologically active polypeptide via a non-peptidyl polymer, can be effectively used for the preparation of a long-acting formulation of protein drugs with remarkably low administration frequency.

## Claims

1. A protein conjugate, in which a physiologically active polypeptide is covalently linked to an immunoglobulin Fc variant *via* a polyethylene glycol,
wherein the immunoglobulin Fc variant has an amino acid sequence represented by SEQ ID NO: 80;
wherein the physiologically active polypeptide is selected from the group consisting of insulin, glucagon, glucagon like peptide-1 and exendin-4;
wherein the immunoglobulin Fc variant shows low FcRn-binding affinity at pH 7.4, but high FcRn-binding affinity at pH 6.0, as compared to the wild-type immunoglobulin Fc fragment; and
wherein the wild-type immunoglobulin Fc fragment is a human aglycosylated IgG4 Fc fragment.

2. The protein conjugate according to claim 1, wherein the wild-type immunoglobulin Fc fragment has an amino acid sequence represented by SEQ ID NO: 73.

3. The protein conjugate according to claim 1, wherein the immunoglobulin Fc variant is produced in animal cells or *E. coli.*

4. A method for preparing the protein conjugate according to claim 1, comprising the step of covalently linking an immunoglobulin Fc variant to a physiologically active polypeptide *via* a polyethylene glycol,
wherein the immunoglobulin Fc variant has an amino acid sequence represented by SEQ ID NO: 80;
wherein the physiologically active polypeptide is selected from the group consisting of insulin, glucagon, glucagon like peptide-1 and exendin-4;
wherein the immunoglobulin Fc variant shows low FcRn-binding affinity at pH 7.4, but high FcRn-binding affinity at pH 6.0, as compared to the wild-type immunoglobulin Fc fragment; and
wherein the wild-type immunoglobulin Fc fragment is a human aglycosylated IgG4 Fc fragment..

## Patentansprüche

1. Proteinkonjugat, in dem ein physiologisch aktives Polypeptid über ein Polyethylenglykol kovalent an eine Immunglobulin-Fc-Variante gebunden ist,
wobei die Immunglobulin-Fc-Variante eine Aminosäuresequenz aufweist, die durch SEQ ID NO: 80 dargestellt ist;
wobei das physiologisch aktive Polypeptid aus der aus Insulin, Glucagon, Glucagon-ähnlichem Peptid-1 und Exendin-4 bestehenden Gruppe ausgewählt ist;
wobei die Immunglobulin-Fc-Variante im Vergleich mit dem Wildtyp-Immunglobulin-Fc-Fragment eine geringe FcRn-Bindungsaffinität bei pH 7,4, aber eine hohe FcRn-Bindungsaffinität bei pH 6,0 aufweist; und
wobei das Wildtyp-Immunglobulin-Fc-Fragment ein menschliches aglykosyliertes IgG4-Fc-Fragment ist.

2. Proteinkonjugat nach Anspruch 1, wobei das Wildtyp-Immunglobulin-Fc-Fragment eine Aminosäuresequenz aufweist, die durch SEQ ID NO: 73 dargestellt ist.

3. Proteinkonjugat nach Anspruch 1, wobei die Immunglobulin-Fc-Variante in tierischen Zellen oder *E. coli* produziert wird.

4. Verfahren zur Herstellung eines Proteinkonjugats nach Anspruch 1, welches den Schritt des kovalenten Bindens einer Immunglobulin-Fc-Variante über ein Polyethylenglykol an ein physiologisch aktives Polypeptid umfasst,
wobei die Immunglobulin-Fc-Variante eine Aminosäuresequenz aufweist, die durch SEQ ID NO: 80 dargestellt ist;
wobei das physiologisch aktive Polypeptid aus der aus Insulin, Glucagon, Glucagon-ähnlichem Peptid-1 und Exendin-4 bestehenden Gruppe ausgewählt ist;
wobei die Immunglobulin-Fc-Variante im Vergleichch mit dem Wildtyp-Immunglobulin-Fc-Fragment eine geringe FcRn-Bindungsaffinität bei pH 7,4, aber eine hohe FcRn-Bindungsaffinität bei pH 6,0 aufweist; und
wobei das Wildtyp-Immunglobulin-Fc-Fragment ein menschliches aglykosyliertes IgG4-Fc-Fragment ist.

## Revendications

1. Conjugué de protéine, dans lequel un polypeptide physiologiquement actif est lié de manière covalente à un variant Fc d'immunoglobuline via un polyéthylèneglycol,
dans lequel le variant Fc d'immunoglobuline présente une séquence d'acides aminés représentée par SEQ ID NO : 80 ;
dans lequel le polypeptide physiologiquement actif est choisi dans le groupe constitué de l'insuline, du glucagon, du peptide-1 analogue ai glucagon et de l'exendine-4 ;
dans lequel le variant Fc d'immunoglobuline présente une faible affinité de liaison à FcRn à un pH de 7,4, mais une affinité de liaison à FcRn élevée à un pH de 6,0, par rapport au fragment Fc d'immunoglobuline de type sauvage ; et
dans lequel le fragment Fc d'immunoglobuline de type sauvage est un fragment Fc d'IgG4 aglycosylée humaine.

2. Conjugué de protéine selon la revendication 1, dans lequel le fragment Fc d'immunoglobuline de type sauvage présente une séquence d'acides aminés représentée par SEQ ID NO : 73.

3. Conjugué de protéine selon la revendication 1, dans lequel le variant Fc d'immunoglobuline est produit dans des cellules animales ou dans E. coli.

4. Procédé de préparation du conjugué de protéine selon la revendication 1, comprenant l'étape de liaison covalente d'un variant Fc d'immunoglobuline à un polypeptide physiologiquement actif via un polyéthylèneglycol,
dans lequel le variant Fc d'immunoglobuline présente une séquence d'acides aminés représentée par SEQ ID NO : 80 ;
dans lequel le polypeptide physiologiquement actif est choisi dans le groupe constitué de l'insuline, du glucagon, du peptide-1 analogue ai glucagon et de l'exendine-4 ;
dans lequel le variant Fc d'immunoglobuline présente une faible affinité de liaison à FcRn à un pH de 7,4, mais une affinité de liaison à FcRn élevée à un pH de 6,0, par rapport au fragment Fc d'immunoglobuline de type sauvage ; et
dans lequel le fragment Fc d'immunoglobuline de type sauvage est un fragment Fc d'IgG4 aglycosylée humaine.
